# EUROPEAN PATENT APPLICATION

(11) **EP 3 332 696 A1**
(43) Date of publication of application: **13.06.2018**
(21) Application number: 17191838.6
(22) Date of filing: 19.09.2017
(51) Int. Cl.: A61B 5/00

(54) **APPARATUS FOR ECG-LIKE WAVEFORM**

(30) Priority: 28.09.2016 US 201615278034
(71) Applicant: Cnoga Medical Ltd., 3060000 Or Akiva (IL)
(72) Inventor: Segman, Yosef, 3060000 Or Akiva (IL)
(74) Representative: Kancelaria Eupatent.pl Sp. z.o.o

(57) **Abstract**

A medical apparatus configured to generate, without multiple electrodes, a signal corresponding to electrical impulses of a heart, and to display a waveform of that signal, comprising: a pulse unit comprising sensor(s) for receiving a single channel pulse signal waveform from a live tissue and for generating a digital pulse signal; a memory buffer; one or more processors configured to constantly apply a derivative function to the digital pulse signal and initiate a display signal to display a waveform of the derivative function during a period of time, wherein the waveform of the derivative function has a shape of an ECG signal waveform of the subject taken during the period of time, wherein the derivative function includes ΔP(t)/Δt, wherein P represents the pulse signal waveform and t is a time variable; and a digital display or printer device configured to display or print the waveform. Multiple channel embodiment also included.

## Description

### FIELD AND BACKGROUND OF THE INVENTION

The present invention is in the field of medical diagnostic devices and methods. More particularly, the present invention aims to provide an ECG-shaped waveform without ECG electrodes.

An electrocardiograph, commonly called an ECG, is a graphic recording of the electrical impulses associated with the heart beat of a subject. It provides very useful diagnostic information. However, it requires fairly complicated equipment to generate and it is typically too bulky to be popular for home use, not only because it usually requires a physician to interpret. Furthermore, it is at least somewhat uncomfortable for a patient to have six electrode leads placed on their chest, which is what is required to obtain a reliable ECG.

The need exists for an apparatus, preferably a portable apparatus, that could be used repeatedly, and noninvasively on patients to obtain an ECG-like reading wherever they are, with minimal effort and discomfort.

### SUMMARY OF THE INVENTION

One aspect of the present invention is a medical apparatus configured to generate, without multiple electrodes, a signal corresponding to electrical impulses of a heart of a mammalian subject, and to display a waveform of that signal, the apparatus comprising a pulse unit comprising at least one sensor for receiving a single channel pulse signal waveform from a live tissue of a mammalian subject and for generating a digital pulse signal during a period of time if the pulse signal waveform was not already digital; a memory buffer for receiving and storing the pulse signal waveform during the period of time; one or more processors configured to constantly apply a derivative function to the digital pulse signal and initiate a display signal to a digital display device to display a waveform of the derivative function during the period of time, wherein the waveform of the derivative function has a shape of an ECG signal waveform of the subject taken during the period of time, wherein the derivative function includes ΔP(t)/Δt, wherein P represents the pulse signal waveform and t is a time variable; and a digital display or printer device configured to display or print the waveform of the derivative function having the shape of the ECG signal waveform.

Another aspect of the present invention is a method of generating, without a plurality of electrodes, a signal that corresponds to electrical impulses of a heart of a mammalian subject, and to display a waveform of that signal, the method comprising utilizing a pulse unit comprising at least one sensor to receive a single channel pulse signal waveform from a live tissue of a mammalian subject and to generate a digital pulse signal during a period of time if the pulse signal waveform was not already digital; storing the digital pulse signal waveform in a computer-readable memory buffer; utilizing one or more processors to constantly apply a derivative function to the digital pulse signal during the period of time as the digital pulse signal is being compiled, wherein a waveform of the derivative function has a set of peaks, the set of peaks shaped like a set of peaks of an ECG signal of the heart of the subject from an ECG taken during the period of time, wherein the derivative function comprises ΔP(t)/Δt, wherein P represents the pulse signal waveform and t is a time variable; initiating a display signal to a digital display device to display the waveform of the derivative function; and displaying, by a digital display device, the waveform of the derivative function.

A still further aspect of the present invention is A medical apparatus configured to generate, a signal corresponding to electrical impulses of a heart of a mammalian subject, and to display a waveform of that signal, the apparatus comprising a pulse unit comprising at least two sensors at different locations of the mammalian subject, wherein each of the at least two sensors is an optical sensor, acoustic sensor, mechanical sensor or magnetic sensor, or any combination thereof, the at least two sensors for receiving multiple channels of pulse signal waveforms from a live tissue of the mammalian subject and for generating, for each channel of the multiple channels, a digital pulse signal during a period of time if the pulse signal waveform was not already digital; a memory buffer for receiving and storing the pulse signal waveform of each channel during the period of time; one or more processors configured to perform one of the following: (i) constantly determine a linear operation in digital pulse signals between any pair of channels of the multiple channels, (ii) apply a derivative operator to any of the digital signal outcomes of the linear operation and (iii) constantly apply a derivative operator to the digital pulse signal of each channel and determine a linear operation between derivative functions of each channel of any of pair of channels, and to initiate a display signal to a digital display device to display a waveform of at least one of (i) the linear operation during the period of time, (ii) the derivative operator during the period of time or (iii) the linear operation on the derivative operator of each channel during the period of time, wherein the waveform of either the derivative function or of the difference between derivative functions of each channel has a shape of at least one kind of ECG signal waveform of the subject taken during the period of time, wherein the derivative function includes ΔP(t)/Δt, wherein P represents the pulse signal waveform and t is a time variable; and a digital display or printer device configured to display or print the waveform of the at least one of (i) the linear operation during the period of time, (ii) the derivative operator during the period of time and (iii) the linear operation on the derivative operator of each channel during the period of time.

These and other features, aspects and advantages of the present invention will become better understood with reference to the following drawings, descriptions and claims.

### BRIEF DESCRIPTION OF THE DRAWINGS

Various embodiments are herein described, by way of example only, with reference to the accompanying drawings, wherein:
Fig. 1 is a schematic illustration of an apparatus using an oximeter as a pulse unit of the apparatus and displaying P, Q, R, S, T peaks, in accordance with one embodiment of the present invention;
Fig. 2 is a schematic illustration of an apparatus using an optical device as a pulse unit of the apparatus and displaying P, Q, R, S, T peaks, in accordance with one embodiment of the present invention;
Fig. 3 is a schematic illustration of a system in which the memory buffer and one or more processors are and remote from the pulse unit and display or printer device and accessible by telecommunications network, in accordance with one embodiment of the present invention;
Fig. 4 is a flowchart showing a method of the present invention;
Fig. 5A is a schematic illustration of multiple channels from multiple sensors of a pulse unit, in accordance with one embodiment of the present invention; and
Fig. 5B is a schematic illustration of an apparatus for multiple channels as in Fig. 5A, in accordance with one embodiment of the present invention.

### DETAILED DESCRIPTION OF THE INVENTION

The following detailed description is of the best currently contemplated modes of carrying out the invention. The description is not to be taken in a limiting sense, but is made merely for the purpose of illustrating the general principles of the invention, since the scope of the invention is best defined by the appended claims.

The present invention generally provides a medical apparatus and method configured to generate, without multiple electrodes, a signal corresponding to electrical impulses of a heart of a mammalian subject, and to display a waveform of that signal. In one example, the apparatus includes a pulse unit. The pulse unit comprises at least one sensor, such as an oximeter, an optical sensor other than an oximeter, a mechanical sensor, an acoustic sensor, an electrode sensor or a magnetic sensor. The apparatus also includes a processing unit, which in one non-limiting example is a processing unit such as that described in US Patent Nos. 8,792,948 or 8,335,550. The processing unit is configured to receive a single channel pulse signal waveform (i.e. an analog pulse signal or a digital pulse signal) from a live tissue of a mammalian subject and generate a digital pulse signal during a period of time if the pulse signal waveform was from an analog signal (i.e. was not already digital). The apparatus also has in some embodiments a memory buffer for receiving and storing real time digital pulse data from the digital pulse signal during the period of time. In some embodiments, one or more processors of the processing unit are configured to constantly apply a derivative function to the digital pulse signal and initiate a display signal to a digital display device to display a waveform of the derivative function during the period of time. Applicant unexpectedly discovered that the waveform of the derivative function has a shape of an ECG signal waveform of the subject that was or would be taken during the period of time. In one embodiment, the derivative function includes ΔP(t)/Δt, wherein P represents the pulse signal waveform and t is a time variable. A digital display device or printer device is configured to receive the display signal and display or print the waveform of the derivative function having the shape of the ECG signal waveform. The memory and one or more processors can be integrated into one physical device or they can be remote from the pulse unit. Anyone at home can obtain a waveform that has the shape of an ECG from a simple portable device without multiple electrodes or technical assistance. A doctor can see the PQRST peaks of the patient's electrical impulses associated with the patient's heart from the display of the apparatus and from the output of the method. In another embodiment, multiple channels are used.

The derivative function may be considered the inner potential within each signal. It has the following shape { ΔP(t)/Δt } e.g. {(P(t+1)-P(t))/ Δt} or {(P(t)-P(t-1))/ Δt}. In the case of the embodiment involving multiple channels, where the processor(s) consider the potential between the reference channel and each of the one or more channels, the derivative function is considering the potential between channels i.e. the potential between Pk and Pj (k ≠ j) without specifically denoting a reference channel. The derivative function is also considering the inner potential of each signal by using the derivative to each channel, i.e. ΔPₖ(t)/Δt for a channel k. If (P₁, P₂,...Pₖ,Pᵣ) is a set of multiple channels where Pr is considered to be the reference channel and all signals are digital or analog signals acquired by non-ECG means, a non-ECG signal may be obtained by a sensor that is not an electrode sensor, for example by an optical sensor (i.e. either a pulse oximeter that uses an electrode or an optical sensor of type described in US Patent Nos. 8,792,948 or 8,335,550 by shining the tissue with light that either traverses the tissue or is reflected from the tissue) to generate an electrical signal analog or digital signal. The multi channel OCG is the synchronized potential (difference) between the channels Pₖ (k=1,2,..K, k≠r) and the reference Pᵣ, between channels (Pₖ and Pₛ for s ≠ k)) and within each channel Pₖ by using DPₖ = ΔPₖ(t)/Δt. The most surprising case is that of the inner potential signal of a single channel where no reference is used and the signal is acquired either optically or electrically, i.e the derivative generates an ECG-like waveform.
In contrast to the prior art, in which obtaining an ECG requires complicated equipment or technical assistance in a medical setting, the present invention generates a display or printout of an ECG-like waveform from a simple device that in some embodiments is portable. The device either requires no electrodes or in the event that the pulse unit sensor is an electrode sensor it requires only one simple electrode.

The principles and operation of an Apparatus and Method for ECG-Like Waveform may be better understood with reference to the drawings and the accompanying description.

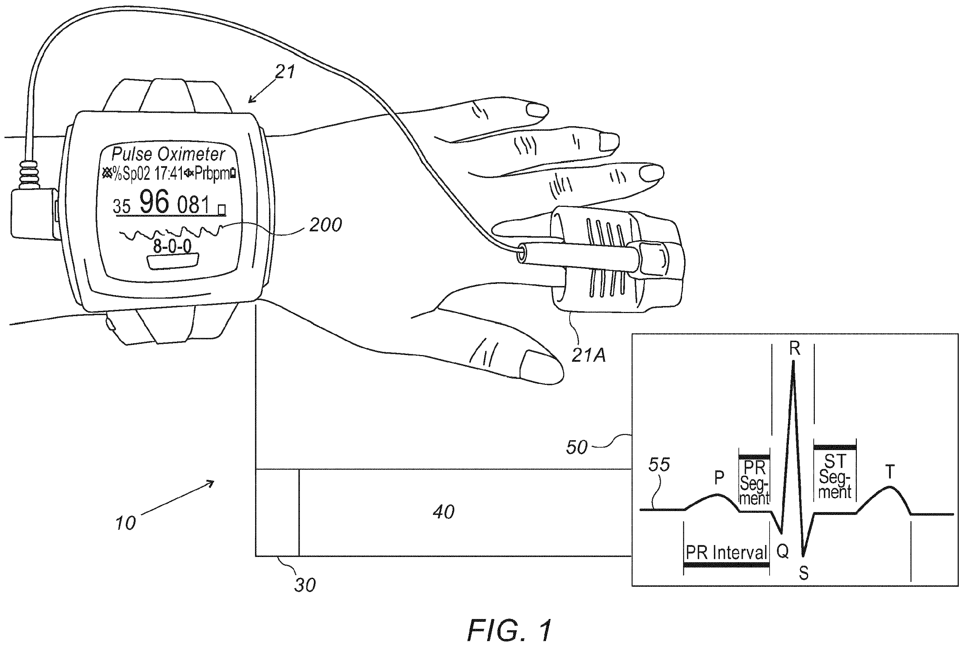

As seen from Figs. 1-2, the present invention, in one embodiment, is a medical apparatus 10 configured to generate, without multiple electrodes, a signal corresponding to electrical impulses of a heart of a mammalian subject, and to display a waveform of that signal. When it is said that the generated signal corresponds to the electrical impulses of the subject's heart, this means that the output of the signal's displayed waveform is similar to the graphic recording of the electrical impulses provided by an actual ECG. It does not mean that the apparatus 10 generates an actual ECG.

The apparatus comprises a pulse unit 20, for receiving a single channel analog or digital pulse signal waveform from a live tissue of a mammalian subject and for generating a digital pulse signal during a period of time, to the extent that the pulse signal waveform is not already a digital pulse signal waveform (i.e. is an analog signal). The pulse unit 20 comprises at least one sensor 21. The at least one sensor is an optical sensor (i.e. an oximeter or an optical sensor of the type described in U.S. Patent Nos. 9,402,546, 8,792,948 and 8,335,550), an electrode sensor, a mechanical sensor, an acoustic sensor or a magnetic sensor or any combination thereof. The term "combination" as used in this patent application, when it refers to combinations of sensors, encompasses (i) a combination of different sensors, including two or more of the multiple types listed here, (ii) a new sensor type that integrates two or more multiple types listed here within one sensor, and (iii) a combination of two or more different sensors that includes the new sensor type mentioned in "(ii)". The term "mechanical sensor" includes, by way of non-limiting example, a microphone and an air tube.

A memory storage component of a computer system, such as a memory buffer 30 of a computer system, is configured to receive and store the pulse signal waveform (for example in real time) during the period of time. In general, the computer system, in all embodiments, includes all necessary hardware and software to perform the functions described herein.

One or more processors 40, which may form an integral device or unit with the pulse unit 20 and memory buffer 30 or may be remote from at least the pulse unit 20, are configured to constantly apply a derivative function to the digital pulse signal and preferably to initiate, compute, transfer, store, display and print the waveform of the derivative function to a digital display device to display the waveform of the derivative function. Preferably the display signal is initiated and outputted during the period of time, for example in one embodiment the one or more processors are configured to initiate the signal to the digital display device while the digital pulse signal is being generated by the pulse unit. The apparatus 10 includes a digital display device 50 or printer device 50 configured to receive the display signal and display or print the waveform of the derivative function having the shape of the ECG signal waveform (acquired by ECG electrodes). In some embodiments, the digital device display 50 is integrated with the pulse unit 20, memory buffer 30 and processor(s) 40 as a single device. In other embodiments, the digital display device 50 is separate.

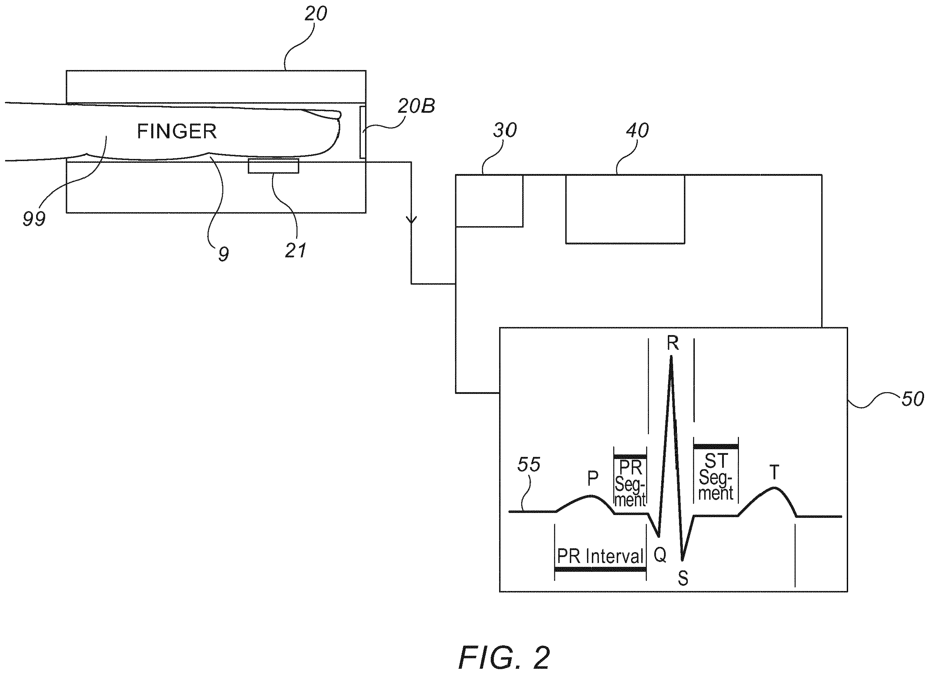

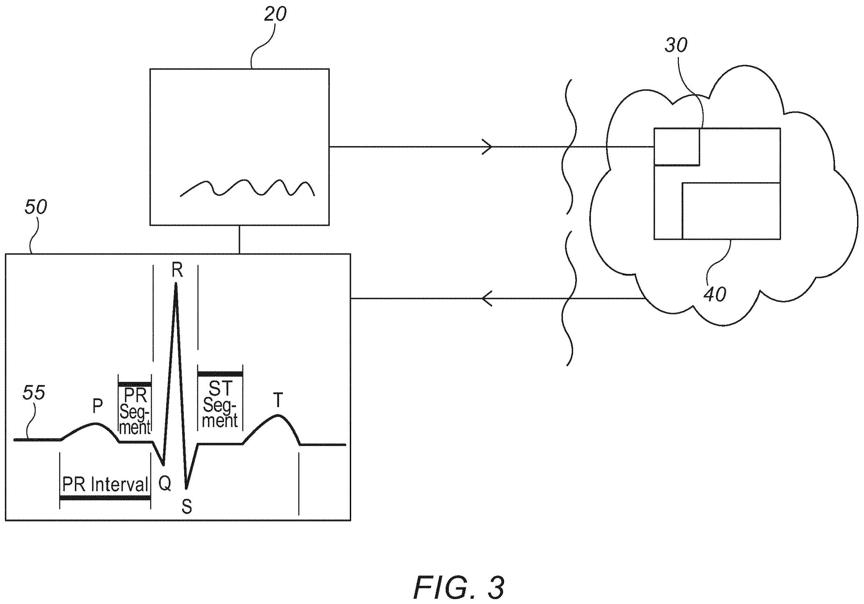

The waveform of the derivative function has a shape of an ECG signal waveform of the subject taken during the period of time. For example, the waveform of the derivative function has a set of peaks that are shaped like a set of peaks of the ECG waveform. In one embodiment shown in Fig. 2 and Fig. 3, the set of peaks of the waveform of the derivative function that are shaped like the set of peaks of the ECG signal waveform comprise "P", "Q", "R", "S" and "T" peaks. One can see the peaks of an ECG signal in the waveform displayed by digital display device 50. In some embodiments, the waveform of the derivative function has a shape that is the same as the ECG waveform from an ECG.

In one embodiment, the derivative function includes ΔP(t)/Δt, wherein P represents the pulse signal waveform and t is a time variable. For example, the derivative function is Y=ΔP(t)/Δt or is Y=-ΔP(t)/Δt or is Y=±ΔP(t)/Δt. In each case P represents the pulse signal waveform and t is a time variable. The derivative function also comprises multiplication by a positive or negative constant in some embodiments. For example, the derivative function is Y=c·ΔP(t)/Δt or is Y=± c·ΔP(t)/Δt, wherein "c" is a positive or negative constant.

The pulse unit 20 preferably comprises at least one sensor 21 that is at least one of an optical sensor, a mechanical sensor, an acoustic sensor, an electrode (electrical) sensor, a magnetic sensor, and a new sensor type that combines any of these types of sensors. The at least one sensor 21 in some embodiments comprises an optical sensor.

As shown in Fig. 2, in the case of an optical sensor other than an oximeter, namely the optical sensor described in U.S. Patent Nos. 9,402,546 8,792,948 or 8,335,550, the pulse unit 20 has a receiving portion 9 configured for receiving a body part 99 of the mammalian subject, in one example a finger 99 of the subject or a distal portion of the finger 99. Fig. 2 shows the pulse unit 20 includes an optical component that comprises at least one sensor, 21, for example at least one digital sensor 21, for receiving a digital image of the body part 99 and the digital image is processed by a processing unit 40 in accordance with U.S. Patent Nos. 9,402,546 8,792,948 or 8,335,550. In that case, it is preferred to utilize the receiving portion of the pulse unit 20 to generate the digital pulse signal at a time that the body part is sufficiently warm, for example in that it has a temperature that is 30°C or greater. Otherwise, the relatively cold temperature would be expected to generate unwanted noise in processing the digital image(s).

Fig. 1 is a schematic illustration of an apparatus 10 using an oximeter as a pulse unit 20 of the apparatus 10. Fig. 1 shows a pulse waveform outputted by an oximeter 21 using an oximeter electrode 21A. The oximeter displays pulse signal waveform 200. The apparatus 10 outputs a waveform 55 of the derivative function having the shape of an ECG signal waveform (i.e. an ECG waveform-like display) that includes P, Q, R, S, T peaks, in accordance with one embodiment. Although the display 50 in Fig. 1 also provides descriptive information designating the P, Q, R, S, T peaks, segments between the peaks or other intervals in addition to the graphic waveform itself, in other embodiments, one or more of this additional descriptive information is not displayed or printed for the user.

In some embodiments, apparatus 10 includes a temperature sensor (not shown) in order to measure the local body temperature identifying local low perfusion. Pulse unit 20 preferably has a mechanism for taking the pulse.

Apparatus 10 is configured to generate the waveform of the derivative function without use of a plurality of electrodes and if the at least one sensor does not include an electrode sensor, in some embodiments without use of any electrodes.

Accordingly, in some embodiments, the apparatus 10 is a medical apparatus configured to generate, without multiple electrodes, a signal corresponding to electrical impulses of a heart of a mammalian subject, and to display a waveform of that signal, the apparatus comprising: a pulse unit 20 comprising at least one sensor 21 for receiving a single channel pulse signal waveform from a live tissue of a mammalian subject and for generating a digital pulse signal during a period of time if the pulse signal waveform was not already digital; a memory buffer 30 for receiving and storing the pulse signal waveform during the period of time; one or more processors 40 configured to constantly apply a derivative function to the digital pulse signal and initiate a display signal to a digital display device to display a waveform of the derivative function during the period of time, wherein the waveform of the derivative function has a shape of an ECG signal waveform of the subject taken during the period of time, wherein the derivative function includes ΔP(t)/Δt, wherein P represents the pulse signal waveform and t is a time variable; and a digital display device 50 or printer device 50 configured to display or print the waveform of the derivative function having the shape of the ECG signal waveform.

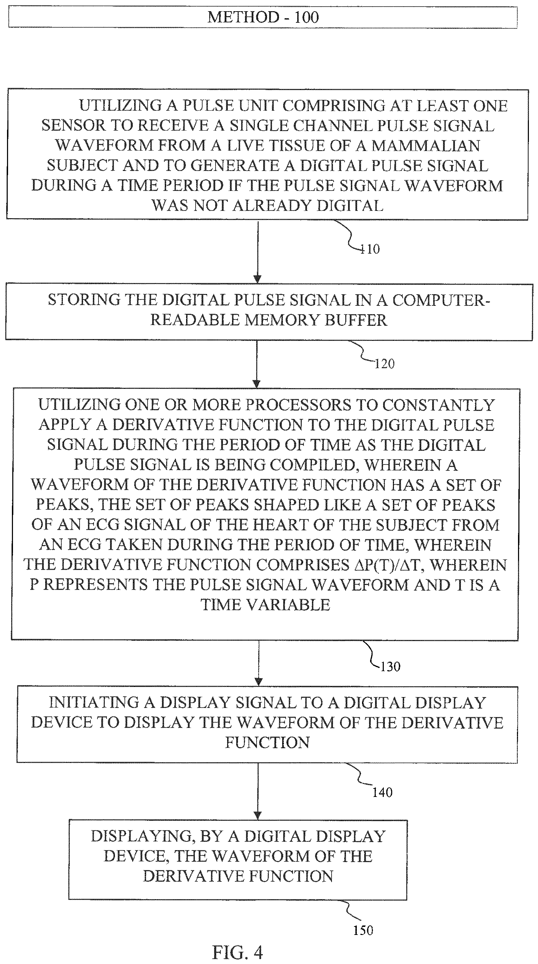

As seen in Fig. 4, a further embodiment of the invention is a method 100 of generating, without multiple electrodes, a signal that corresponds to electrical impulses of a heart of a mammalian subject, and to display a waveform of that signal. The components described in method 100 are as described in relation to apparatus 10. Method 100 comprises a step 110 of utilizing a pulse unit 20 to generate a digital pulse signal during a time period from an analog pulse signal. The pulse unit comprises at least one sensor 21 as in the apparatus 10. This is, in one example, using an oximeter 21. In another example, the pulse unit 20 comprises an optical sensor 21 of the type used in US Patent Nos. 8,792,948 or 8,335,550.

Method 100 also includes a step 120 storing the digital pulse signal in real time in a computer-readable memory buffer 30. Method 100 further includes a step 130 of utilizing one or more processors to repeatedly, or constantly, apply a derivative function to the digital pulse signal during the period of time as the digital pulse signal is being compiled. A waveform of the derivative function has a set of peaks. In one example, the set of peaks are in one embodiment shaped like a set of peaks of an ECG signal of the heart of the subject from an ECG taken during the period of time. For example, the set of peaks shaped like the peaks of the ECG signal comprise "P", "Q", "R", "S" and "T" peaks.

In one embodiment, the derivative function includes ΔP(t)/Δt, wherein P represents the pulse signal waveform and t is a time variable. For example, the derivative function is Y=ΔP(t)/Δt or is Y=-ΔP(t)/Δt or is Y=±ΔP(t)/Δt. In each case P is a variable of the digital pulse signal and t is a time variable. The derivative function also comprises multiplication by a positive or negative constant in some embodiments. For example, the derivative function is Y=c·ΔP(t)/Δt or is Y=± c·ΔP(t)/Δt, wherein "c" is a positive or negative constant.

Method 100 also includes a step 140 of initiating a display signal to a digital display device 50 or printer device 50 to display the waveform of the derivative function and a step 150 of displaying or printing, by a digital display or printer device, the waveform of the derivative function, for example displaying the waveform of the derivative function for the time period.

Another step of method 100 in some cases comprises normalizing the digital pulse signal, for example before (or after) generating the derivative function.

In method 100, the one or more processors and the pulse unit may be utilized as part of one integrated device or alternatively the one or more processors and memory storage component 30 is remote from the pulse unit 20 in some embodiments. In general, the components used in the method 100 of the present invention are the same as in the apparatus 10 of the present invention, and in other embodiments, the same as in apparatus 10A or any other apparatus of the present invention.

The pulse unit 20, as in the apparatus 10 embodiment, may utilize a receiving portion to receive a body part of the mammalian subject. In some steps of method 100 one utilizes the receiving portion of the pulse unit 20 to generate the digital pulse signal at a time that the body part has a temperature, for example a skin temperature at the finger that is 30°C or greater. In accordance with method 100, there is a step in some embodiment of utilizing a receiving portion of the pulse unit to receive a finger of the mammalian subject or utilizing a receiving portion of the pulse unit to receive a body part of the mammalian subject and utilizing at least one digital sensor of the pulse unit to receive a digital image of the body part. Method 100 is in some embodiments carried out without utilizing any electrodes or in some embodiments without utilizing a plurality of electrodes and such that the waveform of the derivative function corresponds to electric impulses of the subject produced in association with the heartbeat of the subject.

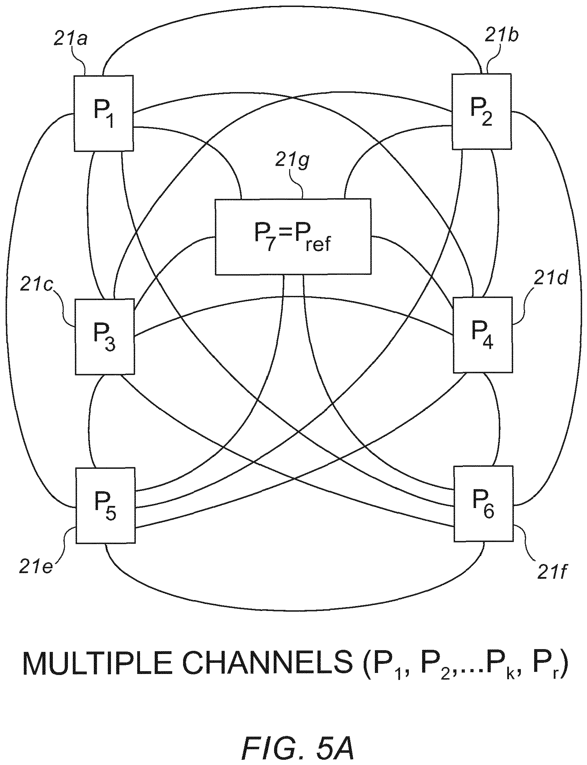

In a further embodiment involving multiple channels shown in Fig. 5A-5B, the present invention is a medical apparatus 10A configured to generate, a signal corresponding to electrical impulses of a heart of a mammalian subject, and to display a waveform of that signal. The idea is the same as in apparatus 10 except that there are multiple channels instead of a single channel and except that the processing by the processing unit 40A processes multiple channels. In addition, this embodiment is for sensors of the pulse unit 20A that do not include electrode sensors. Apparatus 10A comprises: a pulse unit 20 comprising at least two sensors 21A, 21B, etc., which are situated at different locations of the mammalian subject, wherein each of the at least two sensors is an optical sensor, acoustic sensor, mechanical sensor or magnetic sensor, or any combination thereof as that term is defined herein. The at least two sensors are for receiving multiple channels of pulse signal waveforms from a live tissue of the mammalian subject and for generating, for each channel of the multiple channels, a digital pulse signal during a period of time if the pulse signal waveform was not already digital. Apparatus 10A includes a memory buffer 30 is for receiving and storing the pulse signal waveform of each channel during the period of time; one or more processors 40A configured to perform one of the following: (i) constantly determine a linear operation in digital pulse signals between any pair of channels of the multiple channels, (ii) apply a derivative operator to any of the digital signal outcomes of the linear operation and (iii) constantly apply a derivative operator to the digital pulse signal of each channel and determine a linear operation between derivative functions of each channel of any of pair of channels, and to initiate a display signal to a digital display device to display a waveform of at least one of (i) the linear operation during the period of time, (ii) the derivative operator during the period of time or (iii) the linear operation on the derivative operator of each channel during the period of time, wherein the waveform of either the derivative function or of the difference between derivative functions of each channel has a shape of at least one kind of ECG signal waveform of the subject taken during the period of time, wherein the derivative function includes ΔP(t)/Δt, wherein P represents the pulse signal waveform and t is a time variable; and a digital display 50 or printer device 50 configured to display or print the waveform of the linear operation, (ii) derivative operator or of one or more of the linear operations on the derivative operator of each channel.

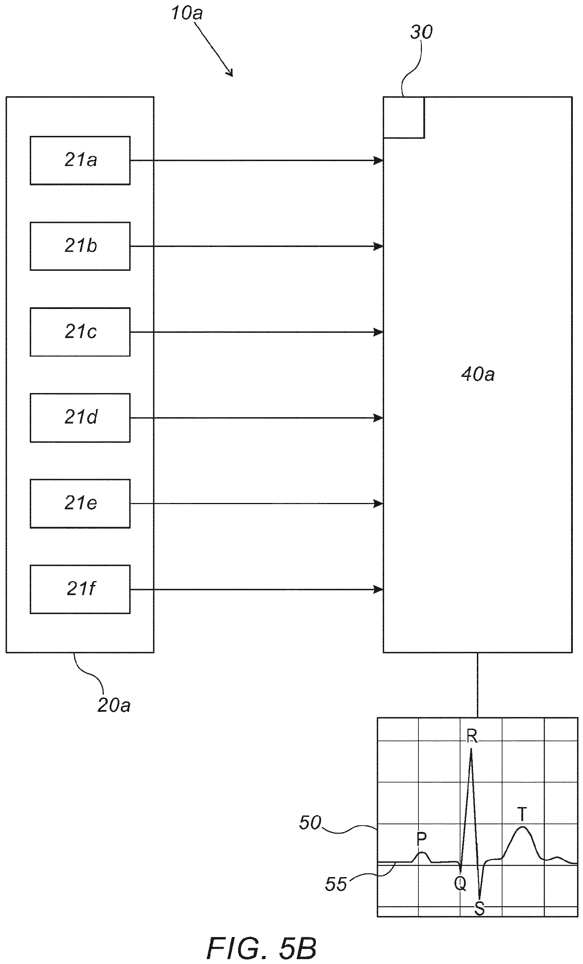

In this case the pulse unit 20 comprises multiple channels derived from multiple sensors, as shown in Fig. 5 including one or more sensors 21a, 21b, 21c ...21n (for example 21a, 21b, 21c ...21g) for receiving one or more analog pulse signals (or digital signals) from a live tissue (for example a finger as in apparatus 10) of a mammalian subject and for generating one or more digital pulse signals (if the pulse signal waveform was not already digital) on one or more channels P₁ through Pₙ during a period of time. In certain embodiments, one of the sensors can be considered a reference sensor 21 on a reference channel P_{R} (for example P₇) during the period of time. The reference digital pulse signal on the reference channel P_{R} is synchronized with the one or more digital pulse signals on the one or more channels P₁ through P_{N} such that the reference channel and the one or more digital pulse signals are sampling the data from the same mammalian subject at the same time.

Apparatus 10A further comprises a memory buffer 30 for receiving and storing real time digital pulse data from the one or more digital pulse signals and the reference digital pulse signal during the period of time. One or more processors 40 are configured to constantly determine one or more potentials between the reference digital pulse signal and each of the one or more digital pulse signals and to initiate one or more display signals respectively to one or more digital display devices to display a waveform of each of the one or more potentials during the period of time. Each waveform of the one or more potentials has a shape of at least one kind of ECG signal waveform of the subject taken during the period of time. When the present invention involves multiple channels, the waveform generated will not show the P, Q, R, S, T peaks shown by the single channel embodiment of the present invention but will show a different ECG-like waveform 55A (Fig. 5B). The particular waveform 55A shown in Fig. 5B is not intended to represent what the different ECG-like waveform 55A will necessarily look like.

Apparatus 10A also comprises one or more digital display devices 50 configured to receive the one or more display signals and display the waveform of each of the one or more potentials, the waveform having the shape of the ECG signal waveform.

While the invention has been described with respect to a limited number of embodiments, it will be appreciated that many variations, modifications and other applications of the invention may be made. Therefore, the claimed invention as recited in the claims that follow is not limited to the embodiments described herein.

## Claims

1. A medical apparatus configured to generate, without multiple electrodes, a signal corresponding to electrical impulses of a heart of a mammalian subject, and to display a waveform of that signal, the apparatus comprising:
- a pulse unit comprising at least one sensor for receiving a single channel pulse signal waveform from a live tissue of a mammalian subject and for generating a digital pulse signal during a period of time if the pulse signal waveform was not already digital;
- a memory buffer for receiving and storing the pulse signal waveform during the period of time;
- one or more processors configured to constantly apply a derivative function to the digital pulse signal and initiate a display signal to a digital display device to display a waveform of the derivative function during the period of time, wherein the waveform of the derivative function has a shape of an ECG signal waveform of the subject taken during the period of time, wherein the derivative function includes ΔP(t)/Δt, wherein P represents the pulse signal waveform and t is a time variable; and
- a digital display or printer device configured to display or print the waveform of the derivative function having the shape of the ECG signal waveform.

2. The apparatus of claim 1, wherein the pulse unit has a receiving portion configured for receiving a body part of the mammalian subject.

3. The apparatus of any one of claims 1-2, wherein the at least one sensor is at least one of an optical sensor, a mechanical sensor, an acoustic sensor, an electrode sensor and a magnetic sensor or any combination thereof.

4. The apparatus of any one of claim 1-3, wherein the pulse unit comprises an optical sensor or a mechanical sensor or any combination thereof.

5. The apparatus of any one of claims 1-4, wherein the pulse unit comprises an electrode sensor.

6. The apparatus of any one of claims 1-5, wherein the waveform of the derivative function includes a set of peaks that are shaped like "P", "Q", "R", "S" and "T" peaks.

7. The apparatus of any one of claims 1-6, wherein the waveform of the derivative function has a shape of an ECG waveform acquired by electrode of an ECG device

8. The apparatus of any one of claims 1-7, wherein the derivative function also comprises multiplication by a positive or negative constant.

9. The apparatus of any one of claims 1-8, wherein the one or more processors are configured to initiate, compute, transfer, store, display and print the waveform of the derivative function.

10. The apparatus of any one of claims 1-9, wherein the apparatus is configured to generate the waveform of the derivative function without use of a plurality of electrodes.

11. The apparatus of any one of claims 1-10, wherein the apparatus is configured to generate the waveform of the derivative function without use of any electrodes.

12. The apparatus of any one of claims 1-11, wherein the at least one sensor is noninvasive.

13. The apparatus of any one of claims 1-12, wherein one or more of the pulse unit, memory buffer, one or more processors and digital display device are situated remotely from at least one other of the pulse unit, memory buffer, one or more processors and digital display device.

14. The apparatus of any one of claims 1-13, further comprising a temperature sensor for sensing a local temperature of the live tissue, wherein the pulse unit is configured to generate the digital pulse signal at a time that the temperature sensor senses the local temperature of the live tissue.

15. A medical apparatus configured to generate, a signal corresponding to electrical impulses of a heart of a mammalian subject, and to display a waveform of that signal, the apparatus comprising:
- a pulse unit comprising at least two sensors at different locations of the mammalian subject, wherein each of the at least two sensors is an optical sensor, acoustic sensor, mechanical sensor or magnetic sensor, or any combination thereof, the at least two sensors for receiving multiple channels of pulse signal waveforms from a live tissue of the mammalian subject and for generating, for each channel of the multiple channels, a digital pulse signal during a period of time if the pulse signal waveform was not already digital;
- a memory buffer for receiving and storing the pulse signal waveform of each channel during the period of time;
- one or more processors configured to perform one of the following: (i) constantly determine a linear operation in digital pulse signals between any pair of channels of the multiple channels, (ii) apply a derivative operator to any of the digital signal outcomes of the linear operation and (iii) constantly apply a derivative operator to the digital pulse signal of each channel and determine a linear operation between derivative functions of each channel of any of pair of channels, and to initiate a display signal to a digital display device to display a waveform of at least one of (i) the linear operation during the period of time, (ii) the derivative operator during the period of time or (iii) the linear operation on the derivative operator of each channel during the period of time, wherein the waveform of either the derivative function or of the difference between derivative functions of each channel has a shape of at least one kind of ECG signal waveform of the subject taken during the period of time, wherein the derivative function includes ΔP(t)/Δt, wherein P represents the pulse signal waveform and t is a time variable; and
- a digital display or printer device configured to display or print the waveform of the at least one of (i) the linear operation during the period of time, (ii) the derivative operator during the period of time and (iii) the linear operation on the derivative operator of each channel during the period of time.
